# FASCICULE DE BREVET EUROPEEN

(11) **EP 3 831 431 B1**
(45) Date de publication et mention de la délivrance du brevet: **25.09.2024**
(21) Numéro de dépôt: 20211158.9
(22) Date de dépôt: 02.12.2020
(51) Int. Cl.: A61M 5/32

(54) **DISPOSITIF D'INJECTION DE PRODUIT FLUIDE**
VORRICHTUNG FÜR DIE EINSPRITZUNG EINES FLÜSSIGEN PRODUKTS
DEVICE FOR INJECTING A LIQUID PRODUCT

(30) Priorité: 02.12.2019 FR 1913622
(43) Date de publication de la demande: 09.06.2021
(73) Titulaire: APTAR France SAS, 27110 Le Neubourg (FR)
(72) Inventeur: PETIT, Ludovic, 27110 VITOT (FR)
(74) Mandataire: CAPRI

(56) Documents cités:
- EP-A1- 2 489 385
- WO-A1-2009/114542
- WO-A1-2011/157930
- WO-A1-2016/174249
- US-A1- 2008 051 715

## Description

La présente invention concerne un dispositif d'injection de produit fluide, et plus particulièrement un tel dispositif pourvu d'un dispositif de sécurité.

Les dispositifs de sécurité pour seringue d'injection, notamment les seringues du type pré-remplies, sont bien connus. Les documents FR2922112, WO 2016/174249 A1, WO 2009/114542 A1, WO 2011/157930 A1, EP 2 489 385 A1 et US 2008/051715 A1 décrivent des exemples d'un tel dispositif de l'état de la technique.

Ces dispositifs de sécurité comportent généralement un manchon externe assemblé autour de la seringue, qui est adapté à se déployer en fin d'injection pour recouvrir l'aiguille. Ces dispositifs peuvent être complexes à fabriquer et à assembler, et leur fiabilité n'est pas toujours optimale.

La présente invention a pour but de fournir un dispositif d'injection qui ne reproduit pas les inconvénients susmentionnés.

La présente invention a aussi pour but de fournir un dispositif d'injection qui soit fiable d'utilisation, qui soit sûr et qui empêche tout risque de blessure.

La présente invention a également pour but de fournir un dispositif d'injection qui soit simple et peu coûteux à fabriquer et à assembler.

La présente invention a donc pour objet un dispositif d'injection de produit fluide tel que décrit dans la revendication 1. Des modes de réaliastion avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif d'injection selon un mode de réalisation avantageux, en position avant injection,
la figure 2 est une vue similaire à celle de la figure 1, en cours d'injection,
la figure 3 est une vue similaire à celle de la figure 2, en fin d'injection, avant déclenchement du dispositif de sécurité, et
la figure 4 est une vue similaire à celle de la figure 3, après déclenchement du dispositif de sécurité.

Les termes "proximal" et "distal" se réfèrent à l'aiguille. Les termes "axial" et "radial" se réfèrent à l'axe central longitudinal du dispositif.

Le dispositif d'injection manuelle représenté sur les figures comporte une seringue comprenant un corps 1 définissant un réservoir 1' contenant le produit à injecter, une aiguille 2 fixée audit corps 1 à travers laquelle le produit est distribué et un piston 3 adapté à se déplacer dans ledit corps 1 pour réaliser cette injection.

L'aiguille 2 comporte une pointe d'injection 20 qui est piquée dans la zone à injecter lors de l'utilisation.

Une tige de piston 5 coopère avec ledit piston 3 lors de l'injection pour le déplacer dans le corps 1.

Le corps 1 de la seringue comporte dans sa partie proximale au moins une, de préférence deux fenêtres 6, ainsi qu'une projection radiale 7 s'étendant radialement vers l'extérieur, dont les fonctions seront détaillées ci-après. Le corps 1 peut classiquement être pourvue d'une collerette radiale 9, de préférence à son extrémité distale, sur laquelle l'utilisateur peut prendre appui lorsqu'il actionne la tige de piston 5. Le corps 1 comporte en outre avantageusement un évidement radial interne 8, de préférence à proximité de ladite collerette radiale 9, et dont la fonction sera également détaillée ci-après.

Un second piston 4 est prévu dans le corps 1, le produit fluide à distribuer étant disposé entre le piston 3 et le second piston 4. Ce second piston 4 a pour fonction d'isoler le produit fluide avant utilisation, et il est percé par l'aiguille 2 en début d'actionnement. Ce second piston 4 est également déplaçable axialement dans ledit corps 1 lors de l'actionnement. La présence de ce second piston garantit l'intégrité du produit fluide jusqu'à l'actionnement.

Un manchon 10 est prévu autour de la seringue, en étant déplaçable axialement par rapport à la seringue entre une position non projetée et une position projetée. Ce manchon 10 comporte une partie interne 11 qui s'étend au moins partiellement à l'intérieur du corps 1 et une partie externe 12 qui s'étend à l'extérieur du corps 1.

Avant injection, le manchon 10 est disposé en position non projetée, de manière à exposer la pointe d'injection 20 de l'aiguille 2.

Il est à noter qu'avant utilisation, cette pointe d'injection 20 peut éventuellement être protégée par un capuchon approprié (non représenté), que l'utilisateur retire lorsqu'il souhaite utiliser le dispositif d'injection. Un tel capuchon n'est toutefois pas obligatoire lorsque le produit fluide est, avant actionnement, isolé entre deux pistons 3, 4, comme dans l'exemple représenté sur les dessins.

Après l'injection, ledit manchon 10 est déplacé axialement par rapport au corps 1 vers sa position projetée, dans laquelle il est disposé autour de la pointe d'injection 20 de l'aiguille 2, pour éviter tout risque de blessure avec ladite aiguille 2, formant ainsi un dispositif de sécurité post-injection.

La partie externe 12 contient un ressort 30 coopérant d'une part avec ledit manchon 10 et d'autre part avec une partie du corps 1 de la seringue, notamment la projection radiale 7 prévue à cet effet. Dans la position non projetée du manchon 10, le ressort 30 est comprimé, pour ainsi solliciter axialement ledit manchon 10 vers sa position projetée.

La partie interne 11 comporte au moins une, de préférence deux, pattes radialement déformables 110 qui, dans la position non projetée du manchon 10, sont encliquetées chacune dans une fenêtre respective 6 du corps 1 de la seringue. Cet encliquetage empêche le manchon 10 d'être déplacé vers sa position projetée, malgré la force du ressort 30, et bloque donc ledit manchon 10 dans sa position non projetée.

Le second piston 4 est associé sur son côté proximal à un organe creux 40, avantageusement conique, adapté à coopérer après injection avec lesdites pattes radialement déformables 110 de la partie interne 11 du manchon 10.

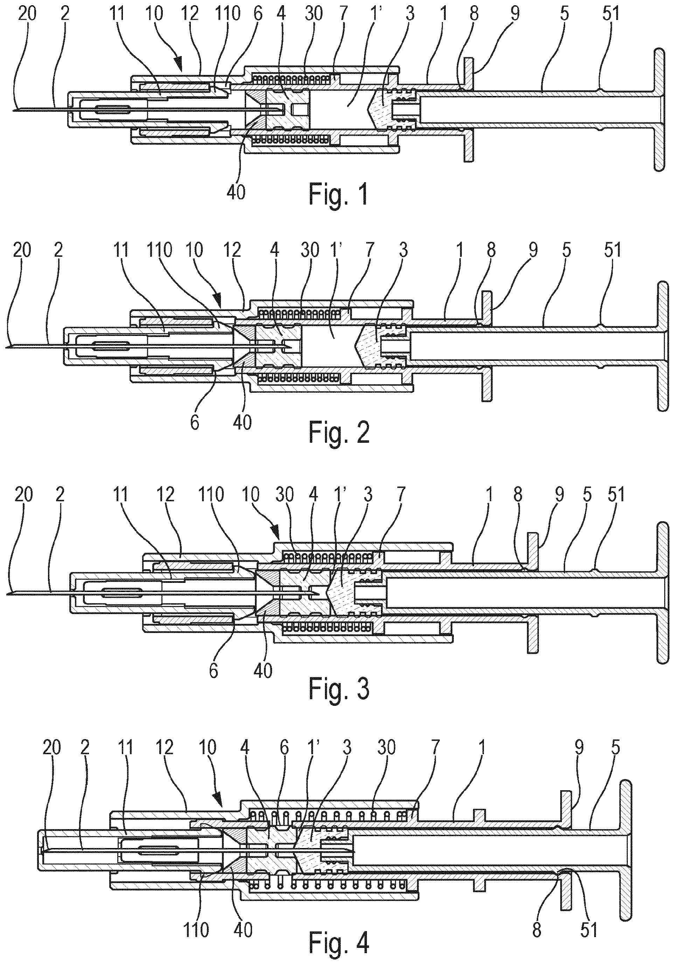

En référence aux figures 1 à 4, l'utilisateur qui souhaite utiliser le dispositif d'injection vient piquer le site d'injection avec la pointe d'injection 20 de l'aiguille 2, puis il exerce une pression axiale sur la tige de piston 5. Celle-ci se déplace axialement par rapport au corps 1, pour déplacer le piston 3 dans le réservoir 1. Ceci génère une pression dans le produit fluide qui, étant incompressible, transmet cette pression au second piston 4. Celui-ci est donc forcé à se déplacer axialement contre l'aiguille 2 qui vient ainsi le percer, comme visible sur la figure 2. A partir de ce moment, le déplacement axial de la tige de piston 5 déplace le piston 3 dans le corps 1, expulsant ainsi le produit fluide contenu dans le réservoir 1' à travers l'aiguille 2 pour l'injecter dans le site d'injection.

En fin d'injection, le piston 3 arrive en butée contre le second piston 4, lui-même en butée contre l'organe creux 40, lui-même en contact de la partie interne 11 du manchon 10, comme visible sur la figure 3. Si l'utilisateur continue son appui axial sur la tige de piston 5, l'organe creux 40 va déformer les pattes radialement déformables 110 vers l'intérieur pour ainsi supprimer leur coopération, notamment leur encliquetage, avec les fenêtres 6 du corps 1. Le manchon 10 n'est alors plus bloqué est alors déplacé automatiquement par le ressort 30 vers sa position projetée, dans laquelle il recouvre la pointe d'injection 20 de l'aiguille 2, pour éviter tout risque de blessure avec l'aiguille 2, comme visible sur la figure 4.

Le déplacement du manchon 10 vers sa position projetée est donc déclenché par un léger déplacement axial de la tige de piston 5, mais est ensuite indépendant de l'utilisateur.

Pour terminer, l'utilisateur déplace manuellement la tige de piston 5 vers une position verrouillée, dans laquelle un bosselage externe 51 de la tige de piston 5 vient coopérer avec l'évidement radial interne 8 du corps 1, pour verrouiller le dispositif dans cette position projetée du manchon 10.

Eventuellement, le manchon 10 pourrait être, en position de repos, relié à la seringue, par exemple par des ponts sécables réalisés par moulage. Ces ponts sécables seraient alors cassés en fin d'injection, lorsque le manchon 10 est déplacé vers sa position projetée. Ainsi, on garantit que lors du déplacement axial de la tige de piston 5 par rapport au réservoir 1, on réalise d'abord l'injection du produit fluide à travers l'aiguille 2 et que le manchon 10 n'est déplacé qu'une fois l'injection terminée. D'autres moyens de liaison du manchon 10 à la seringue en position de repos sont possibles, comme par exemple le frottement ou un coincement mécanique.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif d'injection de produit fluide comportant :
- une seringue comportant un corps (1) définissant un réservoir (1') contenant du produit fluide à injecter, un piston (3) étant disposé axialement déplaçable dans ledit corps (1) et une aiguille (2) pourvue d'une pointe d'injection (20) étant fixée audit corps (1), un second piston (4) étant disposé axialement déplaçable dans le corps (1), ledit produit fluide à injecter étant disposé entre ledit piston (3) et ledit second piston (4),
- une tige de piston (5) déplaçable axialement par rapport audit corps (1) et coopérant avec ledit piston (3) pour le déplacer axialement dans ledit réservoir (1),
- un manchon (10) disposé autour de ladite seringue en étant axialement déplaçable par rapport audit corps (1) entre une position non projetée, dans laquelle ledit manchon (10) ne recouvre pas ladite pointe d'injection (20), et une position projetée, dans laquelle ledit manchon (10) recouvre ladite pointe d'injection (20), ledit manchon (10) étant dans sa position non projetée avant actionnement du dispositif d'injection, et étant sollicité vers sa position projetée par un ressort (30) comprimé en position non projetée,
**caractérisé en ce que** ledit manchon (10) comporte une partie interne (11) qui s'étend au moins partiellement à l'intérieur dudit corps (1) et une partie externe (12) qui s'étend à l'extérieur dudit corps (1), ladite partie interne (11) comportant au moins une patte radialement déformable (110) qui, dans la position non projetée dudit manchon (10), est encliquetée dans au moins une fenêtre respective (6) dudit corps (1), ledit encliquetage bloquant ledit manchon (10) dans sa position non projetée, ledit dispositif comportant un organe creux (40) coopérant avec ledit second piston (4), ledit organe creux (40) étant adapté à coopérer après injection avec ladite au moins une patte radialement déformable (110) pour la déformer radialement vers l'intérieur pour ainsi supprimer sa coopération avec sa fenêtre respective (6) dudit corps (1), de sorte qu'après injection dudit produit fluide, ledit manchon (10) est déplacé automatiquement par ledit ressort (30) vers sa position projetée.

2. Dispositif selon la revendication 1, dans lequel ledit second piston (4) est configuré pour être percé par ladite aiguille (2) en début d'actionnement.

3. Dispositif selon la revendication 1 ou 2, dans lequel ledit organe creux (40) est de forme conique.

4. Dispositif selon l'une quelconque des revendications précédentes, dans lequel ledit ressort (30) coopère d'une part avec ladite partie externe (12) dudit manchon (10) et d'autre part avec une partie dudit corps (1) de la seringue, notamment une projection radiale (7).

5. Dispositif selon l'une quelconque des revendications précédentes, dans lequel ladite tige de piston (5) comporte un bosselage externe (51) coopérant en fin d'actionnement avec un évidement radial interne (8) dudit corps (1), pour verrouiller le dispositif dans la position projetée dudit manchon (10).

## Patentansprüche

1. Vorrichtung zur Injektion eines Fluidprodukts, enthaltend:
- eine Spritze mit einem Körper (1), der ein Reservoir (1') definiert, das das zu injizierende Fluidprodukt enthält, wobei ein Kolben (3) axial verschiebbar in dem Körper (1) angeordnet ist und eine mit einer Injektionsspitze (20) versehene Nadel (2) an den Körper (1) befestigt ist, wobei ein zweiter Kolben (4) axial verschiebbar in dem Körper (1) angeordnet ist, wobei das zu injizierende Fluidprodukt zwischen dem Kolben (3) und dem zweiten Kolben (4) vorgesehen ist,
- eine Kolbenstange (5), die in Bezug auf den Körper (1) axial verschiebbar ist und mit dem Kolben (3) zusammenwirkt, um diesen in dem Reservoir (1) axial zu verschieben,
- eine Hülse (10), die um die Spritze herum angeordnet ist und dabei zwischen einer nicht ausgefahrenen Position, in der die Hülse (10) die Injektionsspitze (20) nicht bedeckt, und einer ausgefahrenen Position, in der die Hülse (10) die Injektionsspitze (20) bedeckt, axial in Bezug auf den Körper (1) verschiebbar ist, wobei sich die Hülse (10) vor der Betätigung der Injektionsvorrichtung in ihrer nicht ausgefahrenen Position befindet und durch eine Feder (30), die in der nicht ausgefahrenen Position komprimiert ist, in ihre ausgefahrene Position beaufschlagt wird,
**dadurch gekennzeichnet, dass** die Hülse (10) einen inneren Abschnitt (11), der sich zumindest teilweise im Inneren des Körpers (1) erstreckt, und einen äußeren Abschnitt (12), der sich außerhalb des Körpers (1) erstreckt, umfasst, wobei der innere Abschnitt (11) zumindest eine radial verformbare Lasche (110) umfasst, die in der nicht ausgefahrenen Position der Hülse (10) in zumindest ein jeweiliges Fenster (6) des Körpers (1) eingerastet ist, wobei durch Einrasten die Hülse (10) in ihrer nicht ausgefahrenen Position gesichert wird, wobei die Vorrichtung ein Hohlorgan (40) umfasst, das mit dem zweiten Kolben (4) zusammenwirkt, wobei das Hohlorgan (40) nach der Injektion mit der zumindest einen radial verformbaren Lasche (110) zusammenwirken kann, um sie radial nach innen zu verformen, um so ihr Zusammenwirken mit ihrem jeweiligen Fenster (6) des Körpers (1) aufzuheben, so dass die Hülse (10) nach der Injektion des Fluidprodukts selbsttätig durch die Feder (30) in ihre ausgefahrene Position verschoben wird.

2. Vorrichtung nach Anspruch 1, wobei der zweite Kolben (4) dazu ausgelegt ist, bei Beginn der Betätigung von der Nadel (2) durchstochen zu werden.

3. Vorrichtung nach Anspruch 1 oder 2, wobei das Hohlorgan (40) konisch ausgeführt ist.

4. Vorrichtung nach einem der vorhergehenden Ansprüche, wobei die Feder (30) einerseits mit dem äußeren Abschnitt (12) der Hülse (10) und andererseits mit einem Abschnitt des Körpers (1) der Spritze, insbesondere einer radialen Nase (7), zusammenwirkt.

5. Vorrichtung nach einem der vorhergehenden Ansprüche, wobei die Kolbenstange (5) eine äußere Erhebung (51) aufweist, die am Ende der Betätigung mit einer radial inneren Aussparung (8) des Körpers (1) zusammenwirkt, um die Vorrichtung in der ausgefahrenen Position der Hülse (10) zu verriegeln.

## Claims

1. A fluid injection device comprising:
- a syringe comprising a body (1) defining a reservoir (1') containing the fluid to be injected, a piston (3) that is arranged to move axially in said body (1), and a needle (2) provided with an injection tip (20) that is fastened to said body (1), a second piston (4) being arranged to move axially in the body (1), said fluid to be injected being arranged between said piston (3) and said second piston (4),
- a piston rod (5) that is axially movable relative to said body (1) and that co-operates with said piston (3) so as to move it axially in said reservoir (1),
- a sleeve (10) that is arranged around said syringe so as to be axially movable relative to said body (1) between a non-projecting position in which said sleeve (10) does not cover said injection tip (20), and a projecting position in which said sleeve (10) covers said injection tip (20), said sleeve (10) being in its non-projecting position before the injection device is actuated, and being urged, while in said non-projecting position, towards its projecting position by a compressed spring (30), the fluid injection device being **characterized in that** said sleeve (10) comprises an inner portion (11) that extends, at least in part, inside said body (1), and an outer portion (12) that extends outside said body (1), said inner portion (11) including at least one radially-deformable tab (110) that, in the non-projecting position of said sleeve (10), is snap-fastened in at least one respective window (6) of said body (1), said snap-fastening blocking said sleeve (10) in its non-projecting position, said device including a hollow member (40) co-operating with said second piston (4), said hollow member (40) being adapted to co-operate after injection, with said at least one radially-deformable tab (110) so as to deform it radially inwards, thereby causing it to cease co-operating with its respective window (6) of said body (1), so that after said fluid has been injected, said sleeve (10) is moved automatically by said spring (30) towards its projecting position.

2. A device according to claim 1, wherein said second piston (4) is perforated by said needle (2) at the start of actuation.

3. A device according to claim 1 or claim 2, wherein said hollow member (40) is of shape that is conical.

4. A device according to any preceding claim, wherein said spring (30) co-operates firstly with said outer portion (12) of said sleeve (10), and secondly with a portion of said body (1) of the syringe, in particular a radial projection (7).

5. A device according to any preceding claim, wherein said piston rod (5) includes an outer bead (51) that, at the end of actuation, co-operates with an inner radial recess (8) of said body (1), so as to lock the device in the projecting position of said sleeve (10).
